# EUROPÄISCHE PATENTANMELDUNG

(11) **EP 2 641 562 A1**
(43) Veröffentlichungstag der Anmeldung: **25.09.2013**
(21) Anmeldenummer: 12194153.8
(22) Anmeldetag: 26.11.2012
(51) Int. Cl.: A61C 5/06, A45D 34/04, A61F 13/40

(54) **Auftragsystem für eine fliessfähige Komponente**

(71) Anmelder: Sulzer Mixpac AG, 9469 Haag (CH)
(72) Erfinder: Müller, Marco, 6030 Ebikon (CH)
(74) Vertreter: Sulzer Management AG

(57) **Zusammenfassung**

Die Erfindung betrifft ein Auftragsystem für eine fliessfähige Komponente.

Die Erfindung geht aus von einem Auftragsystem mit einem Applikator (13) zum Austragen und Auftragen einer Komponente. Die Komponente ist zunächst in einer Vorratskammer (50) nach aussen abgedichtet enthalten. Durch Verschieben einer inneren Hülse (12) in eine äussere Hülse (11) von einer Speicherposition in eine Entnahmeposition gelangt die Komponente in eine mit dem Applikator (13) zugängliche Entnahmekammer (30).

Um ein exaktes Auftragen der Komponente insbesondere an schwer einsehbaren Stellen zu ermöglichen, wird erfindungsgemäss vorgeschlagen, dass das Auftragsystem über eine Schutzhülse (14) verfügt, welche einen Schutzraum ausbildet. Die Schutzhülse (14) ist so ausgeführt und angeordnet, dass sie zumindest einen Teil des Applikators (13) im Schutzraum aufnehmen kann und über sie eine Kraft auf die innere Hülse (12) zum Verschieben der inneren Hülse (12) von der Speicherposition in die Entnahmeposition aufbringbar ist. Damit kann der Applikator (13) sehr filigran ausgeführt werden.

## Beschreibung

Die Erfindung betrifft ein Auftragsystem für eine fliessfähige Komponente gemäß dem Oberbegriff des Anspruchs 1.

In der WO 98/36994 A1 wird ein Auftragsystem für eine fliessfähige Komponente in Form einer Flüssigkeit beschrieben. Das Auftragsystem verfügt über eine äussere Hülse, welche einen äusseren Boden, einen äusseren Mantel und eine äussere Öffnung aufweist, und über eine innere Hülse, welche einen inneren Boden, einen inneren Mantel, eine innere Öffnung und eine Überströmöffnung verfügt. Die innere Hülse bildet eine Entnahmekammer aus, aus welcher die Komponente in einer Entnahmeposition der inneren Hülse mit einem Applikator in Form eines Micropinsels ausgetragen und anschliessend an der gewünschten Stelle aufgetragen werden kann. Die innere Hülse ist zumindest teilweise so innerhalb der äusseren Hülse angeordnet, dass sich in einer Speicherposition der inneren Hülse eine Vorratskammer ausbildet, die durch den äusseren Boden, den inneren Boden und einen Teil des äusseren Mantels begrenzt wird und in welcher die Komponente aufbewahrt werden kann. Durch Verschieben der inneren Hülse aus der Speicherposition in Richtung äusserer Boden in die Entnahmeposition wird über die Überströmöffnung eine Verbindung zwischen der Entnahmekammer und der Vorratskammer hergestellt, so dass die Komponente aus der Vorratskammer in die Austragkammer gelangen kann.

Die Kraft zum Verschieben der inneren Hülse wird über den Applikator aufgebracht. Dazu verfügt der Applikator über eine Auflageschulter, die sich an einem Absatz der inneren Hülse abstützen kann. Damit weist der Applikator zumindest in diesem Bereich einen Durchmesser auf, der dem Innendurchmesser der inneren Hülse entspricht. Ausserdem muss der Applikator so stabil ausgeführt sein und damit einen entsprechenden Durchmesser aufweisen, dass er beim Verschieben der inneren Hülse nicht verbogen wird oder gar bricht.

Demgegenüber ist es die Aufgabe der Erfindung, ein Auftragsystem vorzuschlagen, welches ein exaktes Auftragen der Komponente insbesondere an schwer einsehbaren Stellen ermöglicht. Erfindungsgemäß wird diese Aufgabe mit einem Auftragsystem mit den Merkmalen des Anspruchs 1 gelöst.

Erfindungsgemäss verfügt das Auftragsystem über eine Schutzhülse, welche einen Schutzraum ausbildet. Die Schutzhülse ist so ausgeführt und angeordnet, dass sie zumindest einen Teil des Applikators im Schutzraum aufnehmen kann und über sie eine Kraft auf die innere Hülse zum Verschieben der inneren Hülse von der Speicherposition in die Entnahmeposition aufbringbar ist. Die Verschiebung erfolgt dabei entlang einer Längsachse des Auftragsystems. Die Schutzhülse und die innere Hülse sind damit so ausgeführt, dass sie so miteinander in Kontakt gebracht werden können, dass mittels der Schutzhülse eine Kraft auf die innere Hülse in Richtung äusserem Boden der äusseren Hülse aufgebracht werden kann. Das Verschieben der inneren Hülse erfolgt dabei üblicherweise von Hand.

Der Applikator wird damit nicht zum Verschieben der inneren Hülse genutzt, so dass er nur für das Austragen und Auftragen der Komponente ausgelegt werden kann. Er kann damit sehr filigran ausgeführt werden und muss insbesondere an keiner Stelle einen mit dem Innendurchmesser der inneren Hülse korrespondierenden Abschnitt mit einem vergleichsweise grossen Durchmesser aufweisen. Damit behindert er nur minimal die Sicht auf seine Spitze, über die die Komponente zumeist aufgetragen wird. Dies ist besonders dann vorteilhaft, wenn die Komponente an eine definierte Stelle an einer schwer zugänglichen und einsehbaren Stelle, wie beispielsweise im Mund- oder Rachenbereich oder insbesondere im äusseren Gehörgang des Ohrs aufgetragen werden soll.

Ausserdem ist der Applikator durch die zumindest teilweise Anordnung innerhalb des Schutzraums der Schutzhülse gegen äussere Einflüsse geschützt.

Die fliessfähige Komponente ist insbesondere als eine Flüssigkeit ausgeführt, es ist aber auch möglich eine Paste oder ein Pulver mittels des erfindungsgemässen Auftragsystems aus- und aufzutragen.

Die Hülsen weisen insbesondere einen kreisrunden Querschnitt auf, so dass sie eine hohlzylindrische Grundform aufweisen, die auf einer Seite offen und auf der anderen Seite mit dem Boden verschlossen sind. Der Querschnitt der Hülsen kann aber auch eine andere Form, wie beispielsweise oval, rechteckig oder quadratisch aufweisen. Die innere Hülse kann auch vollständig innerhalb der äusseren Hülse angeordnet sein.

Die Überströmöffnung der inneren Hülse ist insbesondere als Durchgang im inneren Mantel der inneren Hülse ausgeführt. In der Speicherposition ist dann insbesondere zwischen der Überströmöffnung und der Vorratskammer eine Dichtlippe angeordnet, die ein Strömen der Komponente von der Vorratskammer zur Überströmöffnung verhindert. Beim Verschieben der inneren Hülse in die Entnahmeposition wird die genannte Dichtlippe von der Überströmöffnung überfahren und die Komponente kann von der Vorratskammer über die Überströmöffnung in die Austragkammer gelangen. Die Überströmöffnung kann aber auch anders ausgeführt sein. Sie kann beispielsweise als ein Durchgang am inneren Boden der inneren Hülse ausgeführt sein, der in der Entnahmeposition beispielsweise durch eine Folie verschlossen ist. Die Folie kann beispielsweise beim Verschieben der inneren Hülse in die Entnahmeposition durch den sich in der Vorratskammer aufbauenden Druck zerstört und damit die Überströmöffnung geöffnet werden. Es ist auch möglich, dass am äusseren Boden der äusseren Hülse ein in Richtung innerer Boden der inneren Hülse ausgerichteter Dorn angeordnet sein, der die genannte Folie beim Verschieben der inneren Hülse durchsticht und damit zerstört.

Der Applikator weist insbesondere eine längliche, stiftförmige Grundform auf. In einem Kopfbereich sind insbesondere Borsten oder eine Beflockung zur besseren Aufnahme der Komponente beim Austragen angeordnet. Damit weist auch die Schutzhülse insbesondere eine längliche, stiftförmige Grundform auf.

Das Auftragsystem ist insbesondere für das Aus- und Auftragen von nur einer Komponente vorgesehen. Es ist aber auch möglich, dass zwei oder mehr Komponenten vor dem Austragen innerhalb des Auftragsystems gemischt und die gemischten Komponenten ausgetragen werden. Dazu kann beispielsweise in der von der inneren Hülse gebildeten Austragkammer eine erste Komponente vorhanden sein, die nach dem Verschieben der inneren Hülse in die Entnahmeposition mit einer in der Vorratskammer enthaltenen zweiten Komponente vermischt und ausgetragen werden kann. Es kann beispielsweise auch eine dritte Hülse vorgesehen sein, in welcher die äussere Hülse teilweise angeordnet ist. Die äussere Hülse weist dann eine weitere Überströmöffnung auf, wobei die Funktionsweise grundsätzlich gleich ist, wie oben für die Überströmöffnung der inneren Hülse beschrieben. Bei einem derartigen Auftragsystem kann dann in der innerhalb der äusseren Hülse gebildeten Vorratskammer eine erste Komponente und in der innerhalb der dritten Hülse gebildeten weiteren Vorratskammer eine zweite Komponente vorhanden sein. Beim Verschieben der inneren und dann auch der äusseren Hülse in die Entnahmeposition gelangen beiden Komponenten in die Austragkammer und können dort gemischt und anschliessend ausgetragen werden.

Die beiden Hülsen, die Schutzhülse und der Applikator sind beispielsweise aus Polypropylen oder Polyethylen mittels eines Spritzgussverfahrens hergestellt.

Die Vorratskammer ist beispielsweise so dimensioniert, dass sie zwischen 0.05 bis 0.5 ml der Komponenten aufnehmen kann.

In Ausgestaltung der Erfindung ist die Schutzhülse so ausgeführt, dass sie so auf die innere Hülse aufsteckbar ist, dass die Entnahmekammer der inneren

Hülse über die innere Öffnung mit dem Schutzraum der Schutzhülse verbunden ist. Damit kann der Applikator zumindest teilweise innerhalb des sich aus Entnahmekammer und Schutzraum ergebenden Raums angeordnet werden, wodurch er besonders gut geschützt werden kann. Die innere Hülse, der Applikator und die Schutzhülse sind insbesondere so ausgeführt, dass der Applikator vollständig innerhalb der Entnahmekammer und dem Schutzraum der Schutzhülse angeordnet werden kann. Damit kann der gesamte Applikator geschützt in dem genannten Raum angeordnet werden. Die Schutzhülse ist insbesondere an ihrem der inneren Hülse gegenüber liegenden Ende abgeschlossen. Wenn die Schutzhülse auf die innere Hülse aufgesteckt ist, ergibt sich dann aus Entnahmekammer und Schutzraum ein abgeschlossener Raum, in den keine Fremdstoffe eindringen können. Das Auftragsystem stellt damit ein abgeschlossenes System dar, das keine weitere Umverpackung zum Schutz gegen Umwelteinflüsse benötigt. Damit können Verpackungsmaterial und damit Kosten und Entsorgungsaufwand eingespart werden.

In Ausgestaltung der Erfindung weist die innere Hülse am inneren Mantel ein Anschlagelement auf, welches das Aufstecken der Schutzhülse auf die innere Hülse in einer Aufsteckposition beschränkt und über welches eine Kraft in Richtung äusserem Boden auf die innere Hülse aufgebracht werden kann. Damit wird zum einen eine eindeutige Aufsteckposition der Schutzhülse festgelegt und zum anderen kann die Kraft zum Verschieben der inneren Hülse sicher von der Schutzhülse auf die innere Hülse übertragen werden.

Das Anschlagelement ist insbesondere als ein an einer Aussenseite des inneren Mantels entlang umlaufender Bund ausgeführt. Die Schutzhülse weist dann insbesondere einen korrespondierenden umlaufenden Kragen auf. Darunter soll verstanden werden, dass in der Aufsteckposition der Schutzhülle der Kragen über den gesamten Umfang an dem genannten Bund anliegt und sich so eine maximale Berühr- oder Dichtfläche ergibt. Damit kann zum einen die genannte Kraft besonders gut auf die innere Hülse übertragen werden und zum anderen ergibt sich eine besonders sichere Abdichtung der Entnahmekammer und des Schutzraums gegen das Eindringen von Fremdstoffen.

In Ausgestaltung der Erfindung verfügt das Auftragsystem über eine von Schutzhülse und innerer Hülse gebildete Rastierung, mittels welcher die Schutzhülse in der Aufsteckposition auf der inneren Hülse fixierbar ist. Damit kann verhindert werden, dass die Schutzhülle ungewollt die Aufsteckposition verlässt, also von der inneren Hülse herunter rutscht. Somit kann sicher verhindert werden, dass die Entnahmekammer und der Schutzraum ungewollt mit der Umgebung in Kontakt kommen und Schmutz eindringen kann.

Die Rastierung verfügt insbesondere über eine zumindest teilweise, insbesondere vollständig umlaufende Nut, innerhalb welcher in der Aufsteckposition wenigstens eine Ausbuchtung, insbesondere mehrere Ausbuchtungen angeordnet ist. Dabei sind die Nut in einem Teil, also entweder an der inneren Nut oder der Schutzhülle, und die Ausbuchtung am anderen Teil angeordnet. Die Ausbuchtung kann beispielsweise als eine Nase oder ein abschnittsweise oder vollständig umlaufender Steg ausgeführt sein. Damit ist die Rastierung besonders einfach und kostengünstig herstellbar.

In Ausgestaltung der Erfindung weist die Schutzhülse Rippen auf, welche beispielsweise in Quer- und insbesondere in Längsrichtung ausgerichtet sein können. Damit kann die Schutzhülse sehr dünn und damit mit wenig Materialeinsatz und dennoch sehr steif und stabil ausgeführt werden. Damit ist die Schutzhülse besonders kostengünstig herstellbar.

In Ausgestaltung der Erfindung weist die Schutzhülle eine Fixierung des Applikators auf. Unter einer Fixierung soll verstanden werden, dass in der Aufsteckposition der Schutzhülle die Bewegungsmöglichkeit des Applikators innerhalb der Schutzhülle eingeschränkt oder verhindert wird. Dies gilt insbesondere für Bewegungen des Applikators quer zur Längsachse. Damit können Beschädigungen des Applikators beispielsweise während eines Transports vermieden werden. Die Fixierung kann beispielsweise so ausgeführt sein, dass in einem Bereich ein Aussendruchmesser des Applikators und ein Innendurchmesser der Schutzhülse so aufeinander abgestimmt sind, dass sich in der Aufsteckposition der Schutzhülse eine umlaufende Berührfläche zwischen Applikator und Schutzhülse ergibt. Es ist aber beispielsweise auch möglich, dass die Schutzhülse oder der Applikator Vorsprünge aufweisen, die für eine gegenseitige Abstützung sorgen.

In Ausgestaltung der Erfindung ist innerhalb der inneren Öffnung der inneren Hülse einen Führungsring angeordnet. Der Führungsring ist so ausgeführt, dass er den Applikator innerhalb der inneren Öffnung fixiert. Damit wird eine Bewegung des Applikators quer zur Längsachse gegenüber der inneren Hülse und damit eine mögliche Beschädigung des Applikators verhindert. Insbesondere in Kombination mit der genannten Fixierung der Schutzhülle ergibt sich eine besonders sichere Aufbewahrung des Applikators innerhalb des Schutzraums und der Entnahmekammer.

Der Führungsring ist insbesondere so ausgeführt, dass zwischen Führungsring, Applikator und innerer Öffnung ein Durchgang ergibt. Dazu weist der Führungsring beispielsweise eine scheibenförmige Grundform mit einer Öffnung, beispielsweise einem fehlenden Kreissegment auf. Über diesen Durchgang kann der Applikator in den Führungsring eingesetzt und auch wieder entnommen werden.

In Ausgestaltung der Erfindung weist die äussere Hülse eine erste umlaufende Dichtlippe auf, an der die innere Hülse in der Speicherposition ansteht. Damit wird zum einen die Speicherposition der inneren Hülse eindeutig festgelegt und zum anderen wird eine effektive Abdichtung der Vorratskammer erreicht.

In Ausgestaltung der Erfindung weist die äussere Hülse eine zweite umlaufende Dichtlippe auf, welche zwischen der äusseren Öffnung und der Überströmöffnung der inneren Hülse angeordnet ist. Dies gilt sowohl in der Speicherposition, als auch in der Entnahmeposition der inneren Hülse. Damit kann wirkungsvoll verhindert werden, dass Schmutz oder andere unerwünschte Substanzen über den Zwischenraum zwischen innerer und äusserer Hülse über die Überströmöffnung in die Entnahmekammer gelangen können, von wo aus sie mit der Komponenten ausgetragen werden würden. Damit kann während der Lagerung des Auftragssystems ein Eindringen von Fremdstoffen in die Austragkammer wirkungsvoll vermieden werden. Weitere Vorteile, Merkmale und Einzelheiten der Erfindung ergeben sich anhand der nachfolgenden Beschreibung von Ausführungsbeispielen sowie anhand der Zeichnungen, in welchen gleiche oder funktionsgleiche Elemente mit identischen Bezugszeichen versehen sind.

Dabei zeigen:
- Fig. 1: eine Schnittdarstellung eines Auftragsystems,
- Fig. 2: ein mit einem Kreis markiertes Detail des Auftragsystems aus Fig. 1,
- Fig. 3: eine äussere Hülse des Auftragsystems aus Fig. 1,
- Fig. 4: eine innere Hülse des Auftragsystems aus Fig. 1,
- Fig. 5: eine Schutzhülse des Auftragsystems aus Fig. 1,
- Fig. 6: die Schutzhülse aus Fig. 5 in einer perspektivischen Darstellung,
- Fig. 7: einen Führungsring des Auftragsystems aus Fig. 1 und
- Fig. 8: eine äussere Hülse und eine innere Hülse in einer Entnahmeposition.

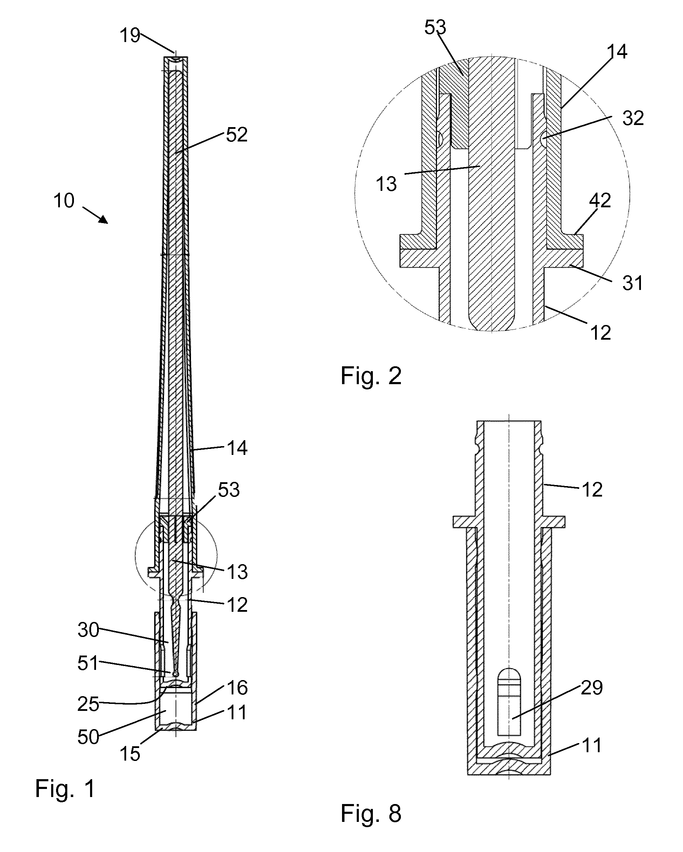

Gemäss Fig. 1 weist ein Auftragssystem 10 eine äussere Hülse 11, eine innere Hülse 12, einen Applikator 13 und eine Schutzhülse 14 auf.

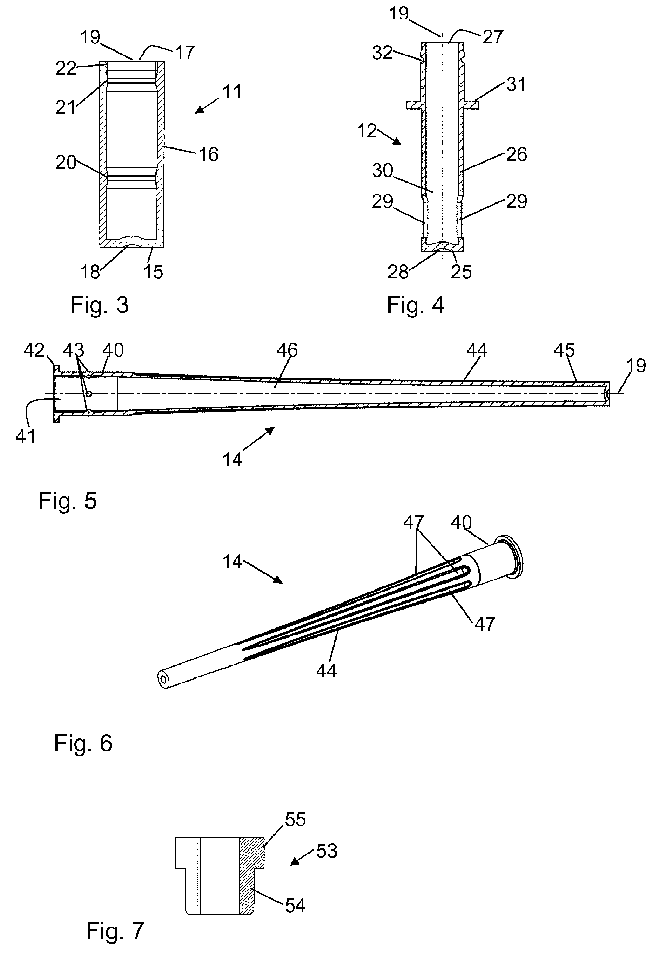

Die äussere Hülse 11, die Fig. 3 als Einzelteil dargestellt ist, weist eine hauptsächlich hohlzylindrische Aussenkontur, und damit einen kreisrunden Aussenquerschnitt auf. Sie verfügt über einen äusseren Boden 15 und einen äusseren Mantel 16, der an einem dem äusseren Boden 15 gegenüberliegenden Ende der äusseren Hülse 11 eine äussere Öffnung 17 ausbildet. Der äussere Boden 15 weist aus fertigungstechnischen Gründen eine erste Einbuchtung 18 nach innen auf, die punktsymmetrisch zu einer Längsachse 19, die auch eine Längsachse des Auftragssystems darstellt, ausgeführt ist.

Der äussere Mantel 16 und damit die äussere Hülse 11 weist eine erste umlaufende Dichtlippe 20 auf. Die erste Dichtlippe 20 ist als ein Abschnitt in Längsrichtung mit um ca. 0.4 bis 1.2 mm kleinerem Innendurchmesser als die angrenzenden Abschnitte ausgeführt, welche denselben Innendurchmesser aufweisen. Der äussere Mantel 16 verfügt ausserdem über eine zweite Dichtlippe 21, die im Bereich der äusseren Öffnung 17 angeordnet ist. Die zweite Dichtlippe 21 ist grundsätzlich gleich ausgeführt wie die erste Dichtlippe 20. Ein Abschnitt 22, der sich zwischen der zweiten Dichtlippe 21 und der äusseren Öffnung 17 erstreckt weist einen grösseren Innendurchmesser auf als ein Abschnitt auf der anderen Seite der zweiten Dichtlippe 21. Damit wird ein Einsetzen der inneren Hülse 12 in die äussere Öffnung 17 der äusseren Hülse 11 erleichtert.

Die innere Hülse 12, die Fig. 4 als Einzelteil dargestellt ist, weist ebenfalls eine hauptsächlich hohlzylindrische Aussenkontur, und damit einen kreisrunden Aussenquerschnitt auf. Sie verfügt über einen inneren Boden 25 und einen inneren Mantel 26, der an einem dem inneren Boden 25 gegenüberliegenden Ende der inneren Hülse 12 eine innere Öffnung 27 ausbildet. Der innere Boden 25 weist eine mit der ersten Einbuchtung 18 des äusseren Boden 15 korrespondierende zweite Einbuchtung 28 nach innen auf.

Der innere Mantel 26 und damit die innere Hülse 12 weisen im Bereich des inneren Bodens 25 zwei sich gegenüber liegende und identisch ausgeführte Überströmöffnungen 29 auf. Die innere Hülse 12 umschliesst eine Entnahmekammer 30.

Der innere Mantel 26 weist an seiner Aussenseite einen als ein nach aussen gerichteter vollständig umlaufender Bund 31 ausgeführtes Anschlagelement auf. Der Bund 31 erstreckt sich dabei senkrecht zur Längsachse 19. Der innere Mantel 26 weist im Bereich der inneren Öffnung 27 ausserdem an seiner Aussenseite eine umlaufende halbkreisförmige Nut 32 auf, die ein erstes Element einer später näher beschriebenen Rastierung bildet, mittels welcher die Schutzhülse 14 in einer in Fig. 1 dargestellten Aufsteckposition, in der die Schutzhülse 14 in einer vorgesehen Position auf die innere Hülse 12 aufgesteckt ist, auf der inneren Hülse 12 fixierbar ist.

Die Schutzhülse 14, die in den Fig. 5 und 6 als Einzelteil dargestellt ist, weist hauptsächlich zwei Bereiche auf. Ein erster Bereich 40 weist eine hauptsächlich hohlzylindrische Aussenkontur auf und verfügt über eine Öffnung 41. Direkt an der Öffnung 41 weist die Schutzhülse 13 einen zum umlaufenden Bund 31 der inneren Hülse 12 korrespondierenden umlaufenden Kragen 42 auf. Die Aussendurchmesser von Bund 31 und Kragen 42 sind damit gleich gross. Ausserdem ist wie in Fig. 2 zu sehen, der Innendurchmesser der Öffnung 41 der Schutzhülse 14 quasi identisch mit dem Aussendurchmesser der inneren Hülse 12 im Bereich des Bunds 31. Der erste Bereich 40 der Schutzhülse 14 weist ausserdem insgesamt vier über den Umfang verteilte Ausbuchtungen 43 auf. Diese Ausbuchtungen 43 bilden ein zweites Element der bereits genannten Rastierung. Sie sind so positioniert, dass sie in der genannten Aufsteckposition der Schutzhülse 14 innerhalb der Nut 32 der inneren Hülse 12 angeordnet sind.

An den ersten Bereich 40 der Schutzhülse 14 schliesst sich ein zweiter Bereich 44 an, über dessen Länge der Durchmesser der Schutzhülse 14 abnimmt und der an einem der Öffnung 41 und damit in der Aufsteckposition der inneren Hülse 12 gegenüber liegenden Ende 45 abgeschlossen ist. Innerhalb der Schutzhülse 14 wird damit ein Schutzraum 46 ausgebildet, der nur die Öffnung 41 aufweist. Der zweite Bereich 44 der Schutzhülle 14 weist mehrere über den Umfang verteilte Rippen 47 auf, die sich in Längsrichtung 19 nicht ganz bis zum Ende 45 der Schutzhülse erstrecken.

In Fig. 1 ist das Auftragsystem 10 in montiertem Zustand dargestellt. Dabei ist die innere Hülse 12 teilweise innerhalb der äusseren Hülse 11 angeordnet. Die innere Hülse 11 ist in einer Speicherposition dargestellt. In dieser Speicherposition ist die innere Hülse 12 so weit in die äussere Hülse 11 eingeschoben, bis sie an der ersten Dichtlippe 20 der äusseren Hülse 11 ansteht. Damit wird innerhalb der äusseren Hülse 11 eine nach aussen abgedichtete Vorratskammer 50 ausgebildet, die durch den äusseren Boden 15, einen Teil des äusseren Mantels 16 der äusseren Hülse 11 und den inneren Boden 25 der inneren Hülse 12 begrenzt wird. In der Vorratskammer 50 wird eine mittels des Auftragsystems 10 aufzutragende Komponente insbesondere in Form einer Flüssigkeit aufbewahrt.

Die Schutzhülse 14 befindet sich in der genannten Aufsteckposition, in der sie so weit auf die innere Hülse 12 aufgesteckt ist, dass der Kragen 42 der Schutzhülse 14 auf dem Bund 31 der inneren Hülse 11 aufliegt. Damit ist der Schutzraum 46 der Schutzhülse 14 über die innere Öffnung 27 mit der Entnahmekammer 30 der inneren Hülse 12 verbunden. In dem dadurch aus Schutzraum 46 und Entnahmekammer 30 entstehenden Raum ist der gesamte Applikator 13 angeordnet. Eine Spitze 51 des Applikators 13 ist dabei innerhalb der Entnahmekammer 30 mit nur geringem Abstand zum inneren Boden 25 angeordnet. Ein der Spitze 51 gegenüber liegender Endbereich 52 des Applikators 13 ist innerhalb des Schutzbereichs 46 der Schutzhülse 14 angeordnet. Ein Aussendurchmesser des Endbereichs 52 des Applikators 13 und ein Innendurchmesser der Schutzhülse 14 sind dabei so gewählt, dass der Applikator 13 im Endbereich 52 an der Schutzhülse 14 anliegt. Damit kann der Applikator 13 innerhalb der Schutzhülle 14 nur entlang der Längsachse 19 bewegen, womit die Schutzhülle 14 über eine Fixierung des Applikators verfügt.

In der inneren Öffnung 27 der inneren Hülse 12 ist ein Führungsring 53 angeordnet, der in Fig. 7 als Einzelteil dargestellt ist. Der Führungsring 53 setzt sich aus zwei hohlzylindrischen Teilen 54, 55 mit identischem Innendurchmesser und unterschiedlichen Aussendurchmessern zusammen. Beiden Teilen 54, 55 fehlt ein Kreissegment, das sich über ca. 100° erstreckt, was in den Figuren allerdings nicht zu sehen ist. Der Aussendurchmesser des im eingebauten Zustand in Richtung äusserer Hülse 11 ausgerichteten Teils 54 entspricht dem Innendurchmesser der inneren Öffnung 27 und der Aussendurchmesser des anderen Teils 55 entspricht dem Aussendurchmesser des inneren Mantels 26 der inneren Hülse 12 im Bereich der inneren Öffnung 27. Der Innendurchmesser des Führungsrings 53 entspricht dem Aussendurchmesser des Applikators 13 im Bereich der inneren Öffnung 27. Damit fixiert der Führungsring 53 den Applikator 13 innerhalb der inneren Öffnung 27, so dass der Applikator 13 sowohl innerhalb der Schutzhülse 14, als auch innerhalb der inneren Öffnung 27 fixiert ist. Die genannten Verhältnisse der Durchmesser der verschiedenen Bauteile sind in Fig. 2 zu sehen.

Soll die Komponente aus der Vorratskammer 50 ausgetragen und aufgetragen werden, so wird die innere Hülse 12 aus der in Fig. 1 dargestellten Speicherposition in eine nicht dargestellte Entnahmeposition verschoben. Dazu wird die äussere Hülse 11 fest gehalten und die Schutzhülse 14 von Hand in Richtung äussere Hülse 11 gedrückt. Damit wird über den Kragen 42 der Schutzhülse 14 eine Kraft auf den Bund 31 und damit auf die innere Hülse 12 in Richtung äusserer Boden 15 eingeleitet. Dadurch wird die innere Hülse 12 über die erste Dichtlippe 20 der äusseren Hülse 11 gedrückt und in Richtung äusserem Boden 15 verschoben. Damit werden auch die Überströmöffnungen 29 in Richtung äusserem Boden 15 und damit in die Entnahmekammer 50 hinein verschoben. Dadurch wird über die Überströmöffnungen 29 eine Verbindung zwischen der Entnahmekammer 30 und der Vorratskammer 50 hergestellt und die Komponente aus der Vorratskammer 50 in die Austragkammer 30 geschoben. Die innere Hülse 12 wird so weit verschoben, bis der innere Boden 25 am äusseren Boden 15 anliegt und damit das gesamte Volumen der Komponente in Richtung Austragkammer 30 gedrückt wurde. Anschliessend kann die Schutzhülse 14 von der inneren Hülse 12 abgenommen werden, so dass der Applikator 13 zugänglich ist und die sich jetzt in der Austragkammer 30 befindliche Komponente mittels des Applikators 13 ausgetragen und an der gewünschten Stelle aufgetragen werden kann. In Fig. 8 ist die innere Hülse 12 innerhalb der äusseren Hülse 11 in Entnahmeposition dargestellt. Die Darstellung in Fig. 8 ist gegenüber den Darstellungen in den Fig. 1 und 4 um 90° gedreht, so dass in Fig. 8 eine Überströmöffnung 29 in der Draufsicht zu sehen ist.

## Patentansprüche

1. Auftragsystem für eine fliessfähige Komponente mit
- einer äusseren Hülse (11), welche einen äusseren Boden (15), einen äusseren Mantel (16) und eine äussere Öffnung (17) aufweist,
- einer inneren Hülse (12), welche einen inneren Boden (25), einen inneren Mantel (26), eine innere Öffnung (27) und eine Überströmöffnung (29) aufweist und eine Entnahmekammer (30 ausbildet und
- einen Applikator (13) zum Austragen und Auftragen der Komponente,
wobei
die innere Hülse (12) zumindest teilweise so innerhalb der äusseren Hülse (11) angeordnet ist, dass
- sich in einer Speicherposition der inneren Hülse (12) eine Vorratskammer (50) ausbildet, die durch den äusseren Boden (15), den inneren Boden (25) und einen Teil des äusseren Mantels (16) begrenzt wird und in welcher die Komponente aufbewahrt werden kann und
- durch Verschieben der inneren Hülse (12) aus der Speicherposition in Richtung äusserer Boden (15) in eine Entnahmeposition über die Überströmöffnung (29) eine Verbindung zwischen der Entnahmekammer (30) und der Vorratskammer (50) hergestellt wird und so die Komponente aus der Vorratskammer (50) in die Austragkammer (30) gelangen kann, aus der sie mit dem Applikator (13) ausgetragen werden kann,
**gekennzeichnet durch**
eine Schutzhülse (14), welche einen Schutzraum (46) ausbildet und so ausgeführt und angeordnet ist, dass sie zumindest einen Teil des Applikators (13) im Schutzraum (46) aufnehmen kann und über sie eine Kraft auf die innere Hülse (12) zum Verschieben von der Speicherposition in die Entnahmeposition aufbringbar ist.

2. Auftragsystem nach Anspruch 1,
**dadurch gekennzeichnet, dass**
die Schutzhülse (14) so ausgeführt ist, dass sie so auf die innere Hülse (12) aufsteckbar ist, dass die Entnahmekammer (30) der inneren Hülse (12) über die innere Öffnung (27) mit dem Schutzraum (46) der Schutzhülse (14) verbunden ist.

3. Auftragsystem nach Anspruch 2,
**dadurch gekennzeichnet, dass**
die innere Hülse (12), der Applikator (13) und die Schutzhülse (14) so ausgeführt sind, dass der Applikator (13) vollständig innerhalb der Entnahmekammer (30) und dem Schutzraum (46) angeordnet werden kann.

4. Auftragsystem nach Anspruch 3,
**dadurch gekennzeichnet, dass**
die Schutzhülse (14) an ihrem der inneren Hülse (12) gegenüber liegenden Ende (45) abgeschlossen ist.

5. Auftragsystem nach einem der Ansprüche 2, 3 oder 4,
**dadurch gekennzeichnet, dass**
die innere Hülse (12) am inneren Mantel (26) ein Anschlagelement (31) aufweist, welches das Aufstecken der Schutzhülse (14) auf die innere Hülse (12) in einer Aufsteckposition beschränkt und über welches eine Kraft in Richtung äusserem Boden (15) auf die innere Hülse (12) aufgebracht werden kann.

6. Auftragsystem nach Anspruch 5,
**dadurch gekennzeichnet, dass**
das Anschlagelement (31) als ein nach aussen gerichteter umlaufender Bund ausgeführt ist.

7. Auftragsystem nach Anspruch 6,
**dadurch gekennzeichnet, dass**
die Schutzhülse (14) einen zum umlaufenden Bund (31) der inneren Hülse (12) korrespondierenden umlaufenden Kragen (42) aufweist.

8. Auftragsystem nach einem der Ansprüche 2 bis 7,
**gekennzeichnet durch**
eine von Schutzhülse (14) und innerer Hülse (12) gebildete Rastierung (31, 43), mittels welcher die Schutzhülse (14) in der Aufsteckposition auf der inneren Hülse (12) fixierbar ist.

9. Auftragsystem nach Anspruch 8,
**dadurch gekennzeichnet, dass**
die Rastierung (31, 43) über eine zumindest teilweise umlaufende Nut (32) verfügt, innerhalb welcher in der Aufsteckposition wenigstens eine Ausbuchtung (43) angeordnet ist.

10. Auftragsystem nach einem der vorhergehenden Ansprüche,
**dadurch gekennzeichnet, dass**
die Schutzhülse (14) Rippen (47) aufweist.

11. Auftragsystem nach einem der vorhergehenden Ansprüche,
**dadurch gekennzeichnet, dass**
die Schutzhülle (14) eine Fixierung des Applikators (13) aufweist.

12. Auftragsystem nach einem der vorhergehenden Ansprüche,
**gekennzeichnet durch**
einen Führungsring (53) der innerhalb der inneren Öffnung (27) der inneren Hülse (12) angeordnet und so ausgeführt ist, dass er den Applikator (13) innerhalb der inneren Öffnung (27) fixiert.

13. Auftragsystem nach Anspruch 12,
**dadurch gekennzeichnet, dass**
der Führungsring (53) so ausgeführt ist, dass zwischen Führungsring (53), Applikator (13) und innerer Öffnung (27) ein Durchgang ergibt.

14. Auftragsystem nach einem der vorhergehenden Ansprüche,
**dadurch gekennzeichnet, dass**
die äussere Hülse (11) eine erste umlaufende Dichtlippe (20) aufweist, an der die innere Hülse (12) in der Speicherposition ansteht.

15. Auftragsystem nach einem der vorhergehenden Ansprüche,
**dadurch gekennzeichnet, dass**
die äussere Hülse (11) eine zweite umlaufende Dichtlippe (21) aufweist, welche zwischen der äusseren Öffnung (17) und der Überströmöffnung (29) der inneren Hülse (12) angeordnet ist.
